(19) **Europäisches Patentamt**
**European Patent Office**
**Office européen des brevets**

(11) **EP 2 675 299 B1**

(12) **EUROPEAN PATENT SPECIFICATION**

(45) Date of publication and mention
of the grant of the patent:
**27.04.2016 Bulletin 2016/17**

(51) Int Cl.:
***A23L 33/17*** *(2016.01)* ***A23J 3/34*** *(2006.01)*

(21) Application number: **12710158.2**

(86) International application number:
**PCT/EP2012/052809**

(22) Date of filing: **17.02.2012**

(87) International publication number:
**WO 2012/110652 (23.08.2012 Gazette 2012/34)**

(54) **A CASEIN HYDROLYSATE**

CASEINHYDROLYSAT

HYDROLYSAT DE CASÉINE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **17.02.2011 EP 11154760**

(43) Date of publication of application:
**25.12.2013 Bulletin 2013/52**

(73) Proprietor: **University of Limerick**
**Limerick (IE)**

(72) Inventors:
• **FITZGERALD, Dick**
**Limerick (IE)**
• **O'SULLIVAN, Dara**
**Listowel**
**Co. Kerry (IE)**

(74) Representative: **Purdy, Hugh Barry**
**PurdyLucey**
**Intellectual Property**
**6-7 Harcourt Terrace**
**D2 Dublin (IE)**

(56) References cited:
**EP-A1- 2 258 208    WO-A1-94/25580**

• **JIANG JINGLI ET AL: "Yak milk casein as a functional ingredient: Preparation and identification of angiotensin-1-converting enzyme inhibitory peptides", JOURNAL OF DAIRY RESEARCH,, vol. 74, no. 1, 1 February 2007 (2007-02-01), pages 18-25, XP009123421,**
• **SPELLMAN D ET AL: "Proteinase and exopeptidase hydrolysis of whey protein: Comparison of the TNBS, OPA and pH stat methods for quantification of degree of hydrolysis", INTERNATIONAL DAIRY JOURNAL, ELSEVIER APPLIED SCIENCE, BARKING, GB, vol. 13, no. 6, 1 January 2003 (2003-01-01), pages 447-453, XP027139899, ISSN: 0958-6946 [retrieved on 2003-01-01]**

EP 2 675 299 B1

**Description**

**Technical Field**

The invention relates to hydrolysates of the milk protein casein, methods of producing the hydrolysates, and uses of the casein hydrolysates as low antigenicity and neutral flavor food ingredients.

**Background to the Invention**

Casein hydrolysates are well known food ingredients and are commonly prepared by hydrolyzing a casein substrate, typically sodium caseinate, with a food grade proteolytic/peptideolytic preparation to a degree of hydrolysis of 20% DH or greater. The high degree of hydrolysis employed heretofore is informed by the requirement to produce a casein hydrolysate which exhibits acceptable organoleptic properties while also providing low antigenicity, the latter being a key requirement for casein hydrolysates intended for use as a food ingredient in infant formula, and foodstuffs for immunocompromised or geriatric individuals. A problem with existing casein hydrolysates, especially those that are extensively hydrolysed, is that the resultant hydrolysate either does not have the required reduction of antigenicity, or that the hydrolysate is bitter, or both. A casein hydrolysate having an acceptable bitterness score as well as greatly reduced antigenicity has eluded the food industry for decades.

Whey hydrolysates have different bitterness characteristics compared to casein hydrolysates. For example, whey hydrolysates prepared at a given degree of hydrolysis would tend to have significantly lower bitterness than casein hydrolysates prepared at the same degree of hydrolysis, especially at a degree of hydrolysis of 20% or lower. FlavorPro-Whey™ proteolytic/peptideolytic preparations (Biocatlaysts Inc) are food grade enzyme preparations sold for the production of whey hydrolysates, and for de-bittering of whey-derived milk products.

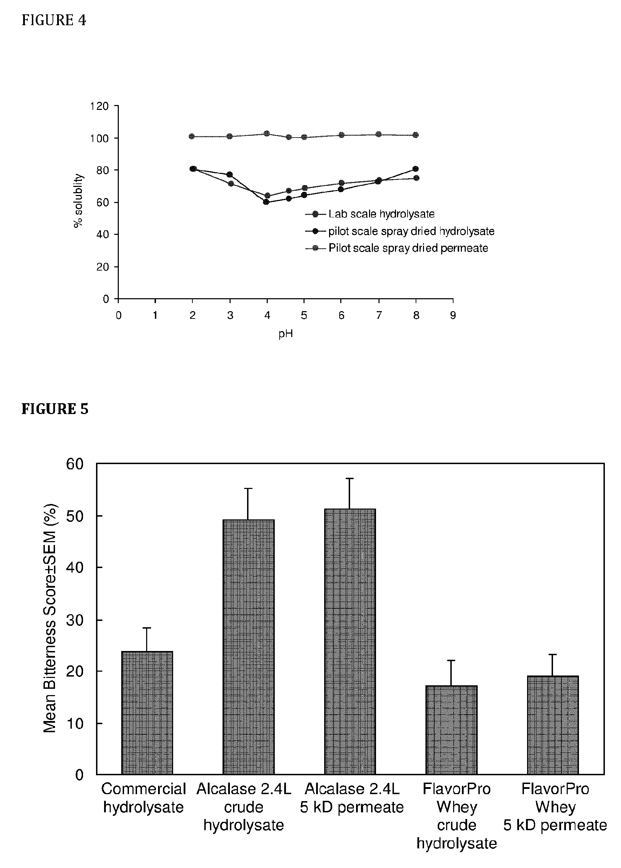

EP2258208 describes a casein hydrolysate formed by hydrolysis of sodium caseinate using Alcalase™ and employing degrees of hydrolysis (%DH) of from 1 to 20 %DH. The hydrolysates formed were found to have greatly reduced antigenicity compared to unhydrolysed casein, but also to have an unacceptably bitter flavor (see comparative example of Figure 5 below). Jingli et al (J. Dairy Res., Vol. 74, No. 1, 2007) discloses a casein hydrolysate from yak milk prepared using Flavourzyme™, a protease from *Aspergillus oryzae,* and specifically describes experiments designed to identify peptides having angiotensin-I-converting enzyme inhibitory activity. WO94/25580 discloses a casein hydrolysate prepared using Flavorzyme™.

It is an object of the invention to overcome at least one of the above-referenced problems for casein hydrolysates.

**Statements of Invention**

According to the invention, there is provided a casein hydrolysate according to Claim 1.

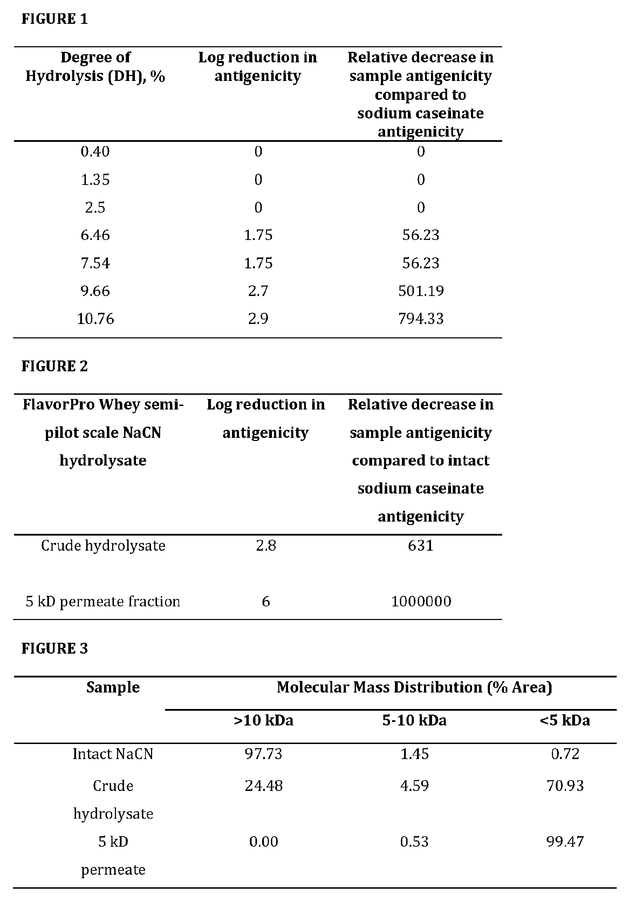

The hydrolysate typically has a relative decrease in antigenicity compared to intact sodium caseinate, preferably of at least 50, 100, 200, 300, 400, 450, 500, 600, 700, or 750 fold. The term "relative decrease in antigenicity" means a reduction in antigenicity as determined using an ELISA protocol employing polyclonal antisera raised in rabbit using the method described below. For example, and referring to Figure 1, a 56.23 fold decrease in antigenicity correlates to a 98.22% reduction in antigenicity of the sample (hydrolysate) compared to that of the reference (intact sodium caseinate). Thus, the hydrolysate has at least a 98%, 98.2%, 98.4%, 98.6%, 98.8%, 99%, 99.2%, 99.4%, 99.6%, 99.8% or 99.9% reduction in antigenicity compared to intact sodium caseinate.

In another embodiment, the hydrolysate typically has a log reduction in antigenicity compared to intact sodium caseinate of at least 1.5, 1.7, 2.5, 2.7, 2.8 or 2.9. The term "log reduction in antigenicity" means the reduction in antigenicity as determined using an ELISA protocol employing polyclonal antisera raised in rabbit using the method described below. For example, and referring to Figure 1, a log reduction in antigenicity of 1.75 correlates to a 98.22% reduction in antigenicity of the sample (hydrolysate) compared to that of the reference (intact sodium caseinate).

The casein hydrolysate has a mean bitterness score of less than 30% as determined using the bitterness scoring method described below in which a 100% bitterness score correlates with 1g/L aqueous caffeine solution and a 0% bitterness score correlates with Ballygowan™ Still Mineral Water. Ideally, the hydrolysate has a mean bitterness score of less than 29%, 28%, 27%, 26%, 25%, 24%, 23%, 22%, 21%, 20%, 19%, or 18%.

Casein hydrolysates of the invention have been found to have reduced antigenicity as well as a low bitterness score. This is surprising as it would have been unexpected that hydrolysis of casein to a %DH of 15% or less would provide a casein hydrolysate having the low levels of antigenicity observed, while also having a neutral flavor (See Fig. 1 and Fig. 5 below). Without being bound by theory, it is believed that these attributes are due to the choice of an aspergillus-derived proteolytic preparation, which heretofore has been employed for production of whey hydrolysates, and the relatively low %DH.

The invention also relates to a casein hydrolysate according to Claim 1that is further characterized in that it comprises of peptides spread over the following distribution, as a function of their molecular mass:

>10kD - 0 to 30%
5-10kD - 0.1 to 8%
<5kD - 60 to 99.7%.

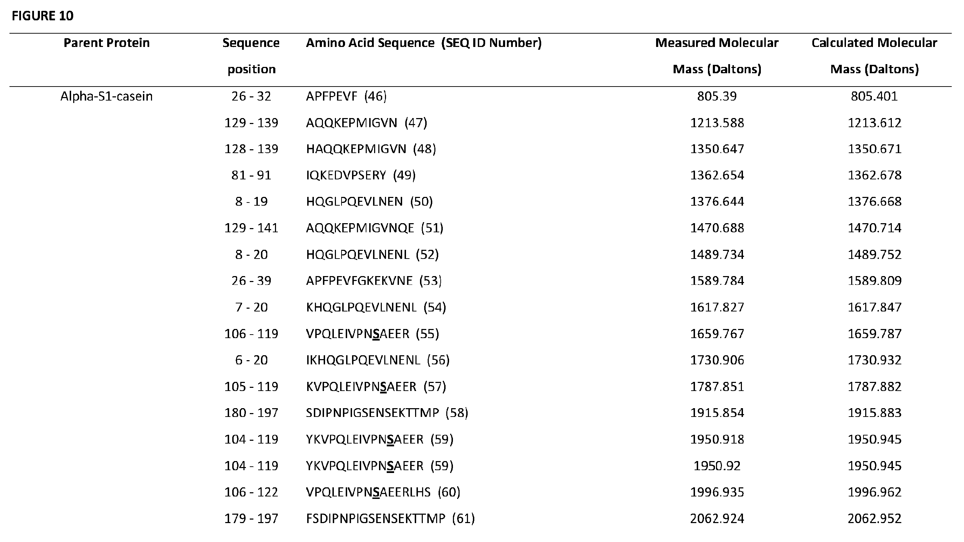

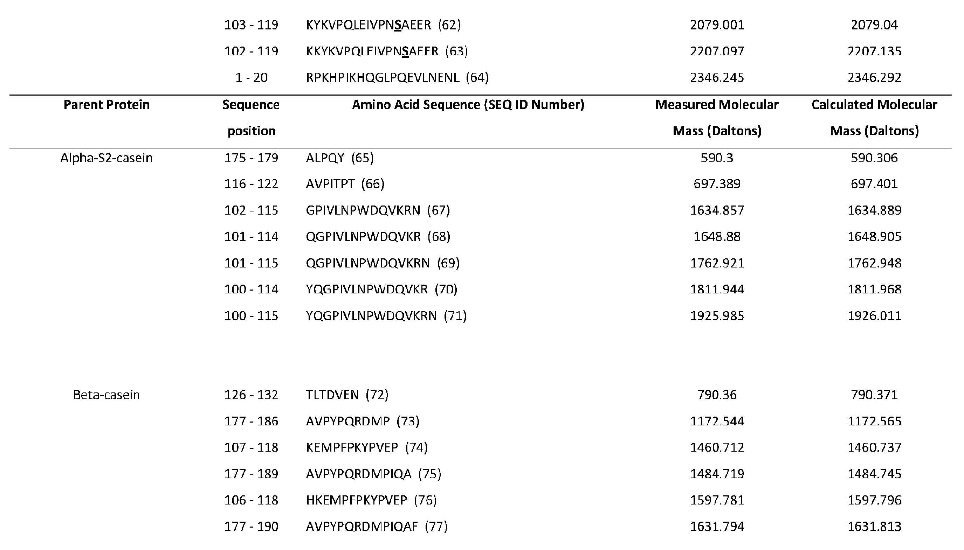

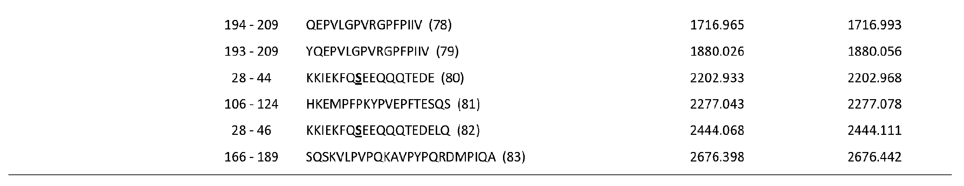

The invention also relates to a casein hydrolysate according to Claim 1 having a peptide profile substantially similar to that shown in Fig. 10, or comprising substantially all of the peptides shown in Fig. 10. In this regard, the term "substantially similar" or "comprising substantially all" should be understood to mean comprising at least 80%, 85%, 90%, 91%, 92%, 93%, 94%, 95%, 96%, 97%, 98%, 99% or 100% of the casein peptides of Figure 10 - SEQUENCE ID NO's 46 to 88 (for example, comprising 35 of the 43 peptides).

The invention also provides a low molecular weight fraction of a casein hydrolysate of Claim 5.Surprisingly, it has been found that removal of the mid- and high-MW peptide and protein fraction decreases the antigenicity of the permeate compared to the crude hydrolysate by up to a thousand-fold, while maintaining the low bitterness characteristics of the crude hydrolysate. See for example Fig. 2 and Fig. 5 below. The low molecular weight fraction is obtainable by preparing a crude hydrolysate according to the invention, and then separating out the desired range of peptides/proteins, for example by ultrafiltration.

Ideally the low molecular weight fraction is further characterized in that it comprises of peptides spread over the following distribution, as a function of their molecular mass:

>10kD - 0 to 1%, ideally 0 to 0.1%
5-10kD - 0.1 to 1%, ideally 0 to 0.9%
<5kD - 98 to 99.9%, ideally 99 to 99.9%.

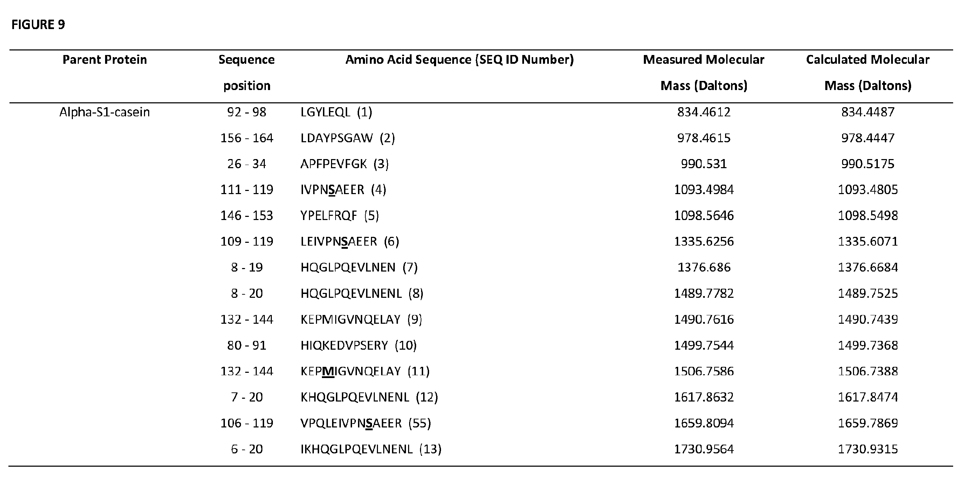

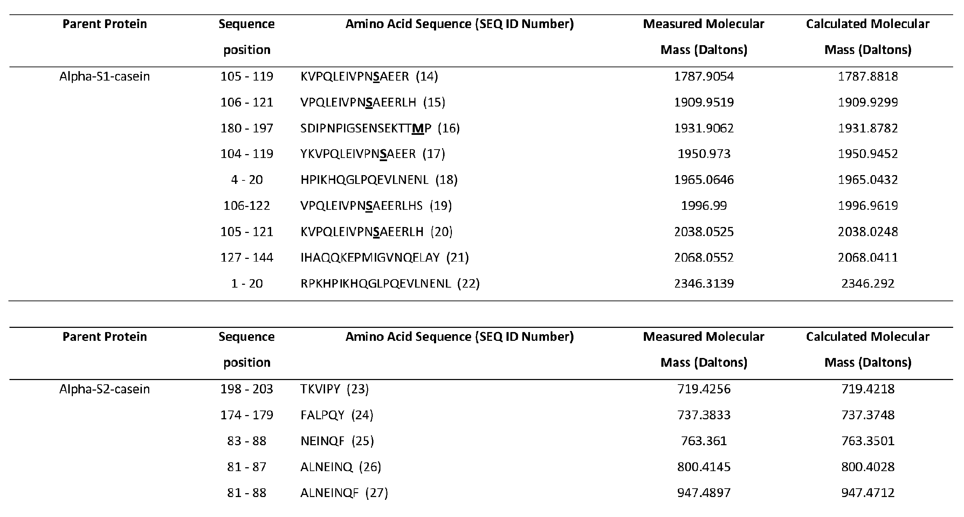

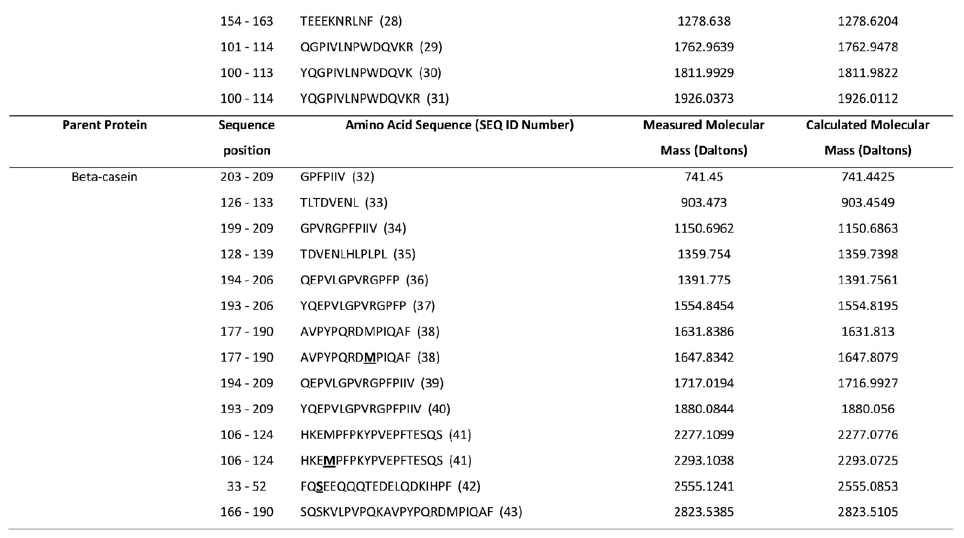

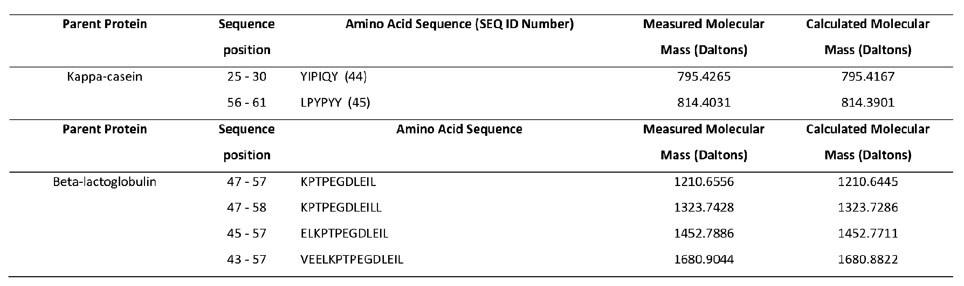

The invention also relates to a low molecular weight fraction of the casein hydrolysate of Claim 5 having a peptide profile substantially similar to that shown in Fig. 9, or comprising substantially all of the peptides shown in Fig. 9. In this regard, the term "substantially similar" or "comprising substantially all" should be understood to mean comprising at least 80%, 85%, 90%, 91%, 92%, 93%, 94%, 95%, 96%, 97%, 98%, 99% or 100% of the casein peptides of Figure 9 - SEQUENCE ID NO's 1 to 46 and 55 (for example, comprising 40 of the 47 casein-derived peptides).

The invention also relates to an infant formula in solid (i.e. powder, flakes, particulates) or liquid form comprising a casein hydrolysate, or low molecular weight fraction thereof, of the invention, optionally further including one or more of a carbohydrate source (e.g., lactose), vitamins, minerals, and other food protein hydrolysates. The formulation of infant formula will be well known to those skilled in the art, and is described in EP0631731 and WO2006/130204.

The invention also relates to the use of a casein hydrolysate, or low molecular weight fraction thereof, of the invention in the formulation of a foodstuff for infants, geriatrics, or immunocompromised individuals, or a sports nutritional foodstuff.

The invention also relates to a food ingredient, especially a food ingredient having low antigenicity and non-bitter flavour, comprising a casein hydrolysate, or low molecular weight fraction thereof, of the invention.

The invention also relates to a method for producing a casein hydrolysate according to the invention, the method comprising the steps of hydrolyzing a casein substrate to a degree of hydrolysis of from 5%DH to 15%DH, ideally of from 9%DH to 15%DH using an *aspergillus*-derived proteolytic formulation, for example an *aspergillus*-derived proteolytic preparation including *aspergillus*-derived serine protease and an *aspergillus*-derived calcium-dependent protease, ideally a FlavorPro-Whey™ proteolytic/peptidolytic formulation.

The invention also relates to a method for producing a low molecular weight fraction of a casein hydrolysate according to the invention, the method comprising the steps of hydrolyzing a casein substrate to a degree of hydrolysis of from 5%DH to 15%DH using an *aspergillus*-derived proteolytic formulation, for example an *aspergillus*-derived proteolytic preparation including an *aspergillus*-derived serine protease and *aspergillus*-derived calcium-dependent protease, ideally a FlavorPro-Whey™ protease formulation, and fractionating the hydrolysate using separation means to provide a low molecular weight fraction which is substantially free of peptides having a MW of greater than 5kDa.

## Brief Description of the Figures

Fig. 1 is Table showing antigenicity values of a series of lab-scale casein hydrolysates, including four casein hydrolysates of the invention - a% sample residual antigenicity calculated by dividing intact sodium caseinate antigenicity (given a nominal value of 1) by relative decrease in sample antigenicity (column 3) and multiplying the resulting fraction by 100 to give the % residual activity remaining in the sample compared to that of intact NaCN. The % decrease in antigenicity was obtained by subtracting the % residual activity from 100 % (Antigenicity of intact NaCN).
EXAMPLE - 6.46 % DH sample

$$\% \text{ sample residual antigenicity} = (1/56.23) \text{ X } 100\% = 1.7784\% \approx 1.78\%$$

% reduction in sample antigenicity
compared to antigenicity of intact NaCN= 100 % - 1.78 %= 98.22 %
i.e. 98.22 % of the antigenicity has been removed = 1.75 log reduction = 56.23 relative decrease;

Fig. 2 is a Table comparing antigenicity values of a casein hydrolysate of the invention (Crude Hydrolysate - 14.16%DH) with a low molecular weight fraction of the Crude Hydrolysate (5 kD permeate fraction) - [a] % sample residual antigenicity calculated by dividing intact sodium caseinate antigenicity (given a nominal value of 1) by relative decrease in sample antigenicity (column 3) and multiplying the resulting fraction by 100 to give the % residual activity remaining in the sample compared to that of intact NaCN
The % decrease in antigenicity was obtained by subtracting the % residual activity from 100 % (Antigenicity of intact NaCN)
EXAMPLE - 5 kD permeate fraction

$$\% \text{ sample residual antigenicity} = (1/1000000) \text{ X } 100\% = 0.0001\%$$

% reduction in sample antigenicity
compared to antigenicity of intact NaCN= 100 % - 0.0001 %= 99.9999 %
i.e. 99.9999 % of the antigenicity has been removed = 6 log reduction = 1,000,000 relative decrease.

Fig. 3 is a Table showing the molecular mass distribution profile of intact sodium caseinate, a casein hydrolysate of the invention (Crude Hydrolysate - 14.16%DH), and a low molecular weight fraction of the Crude Hydrolysate (5kD permeate);

Fig. 4 is a Table showing the nitrogen solubility indices of a lab scale casein hydrolysate of the invention, a pilot scale casein hydrolysate of the invention, and a low molecular weight fraction (5kD permeate) of the pilot scale casein hydrolysate;

Fig. 5 is a graph comparing the Mean Bitterness Scores of (a) a commercial casein hydrolysate (comparative), (b) an Alcalase™ casein hydrolysate (comparative), (c) a low molecular weight fraction of the Alcalase™ casein hydrolysate (comparative), (d) a casein hydrolysate of the invention (14.16%DH), and (c) a low molecular weight fraction of the casein hydrolysate of the invention;

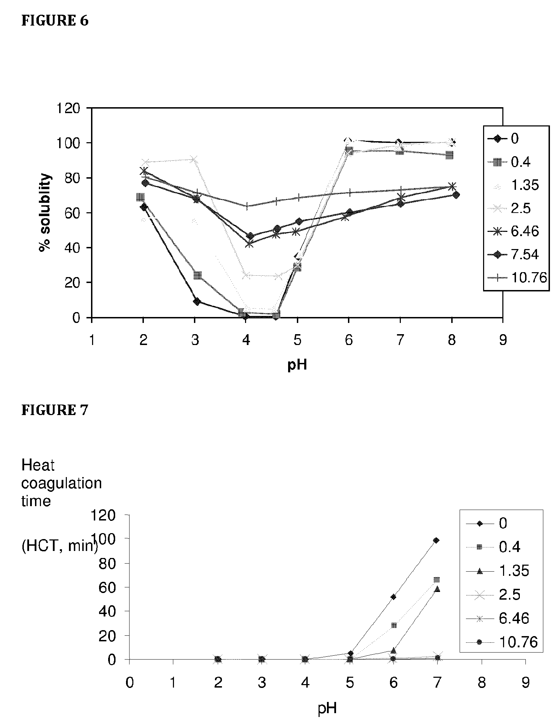

Fig. 6 is a graph showing the Nitrogen Solubility Index of sodium caseinate hydrolysates generated with FlavorPro Whey™ protease preparations at different %DH values as a function of pH;

Fig. 7 is a graph showing Heat Coagulation Time (HCT) of sodium caseinate hydrolysates generated by FlavorPro Whey™ at different %DH values as a function of pH;

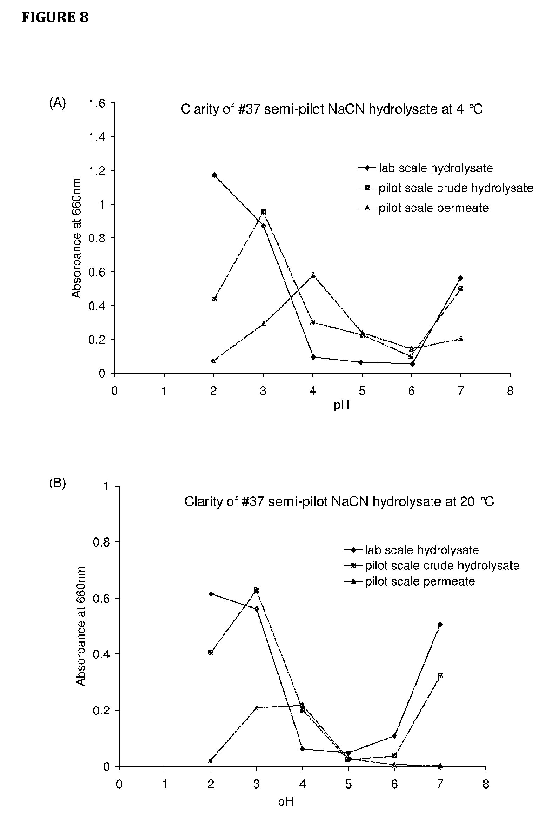

Fig. 8A and 8B are Clarity Profiles at 660nm and 4°C (Fig. 8A) and 20°C (Fig. 8B) of (a) semi-pilot scale FlavorPro Whey™ generated crude hydrolysate (14.16%DH), (b) a low molecular weight fraction (5kD permeate) of (a), and (c) lab scale FlavorPro Whey™ generated crude hydrolysate (10.76%DH);

Fig. 9 is a Table showing the sequences and molecular weight profile of peptides in a low molecular weight fraction (<5kD) of a FlavorPro Whey™ generated 14.16%DH casein hydrolysate of the invention in which the Alpha S1 peptides 1 to 23 correlate with SEQUENCE ID NO'S 1 to 22 and 55, Alpha S2 casein peptides 1 to 9 correlate with SEQUENCE ID NO'S 23 to 31, Beta casein peptides 1 to 12 correlate with SEQUENCE ID NO'S 32 to 43, and Kappa casein peptides 1 and 2 correlate with SEQUENCE ID NO's 44 and 45;

Fig. 10 is a Table showing the sequences and molecular weight profile of peptides in a FlavorPro Whey™ generated 10.76%DH casein hydrolysate of the invention in which the Alpha S1 peptides 1 to 19 correlate with SEQUENCE ID NO'S 46 to 64, Alpha S2 casein peptides 1 to 7 correlate with SEQUENCE ID NO'S 65 to 71, and Beta casein peptides 1 to 12 correlate with SEQUENCE ID NO'S 72 to 83.

## Detailed Description of the Invention

The invention provides a casein hydrolysate, and its use as a food ingredient, typically but not limited to foods such as infant formula, geriatric food products, sports and nutritional foods and supplements, beverages, and food products for immunocompromised individuals. The casein hydrolysate is formed by controlled hydrolysis of a casein substrate (for example, to a degree of hydrolysis of about 5%DH to 15%DH) using a proteolytic/peptideolytic formulation derived from an *aspergillus* fungus. Ideally, the controlled hydrolysis employs a FlavorPro-Whey™ proteolytic formulation. The molecular weight profile of the peptides in a casein hydrolysate of the invention is shown in Fig. 10 and the Sequence Listing appended hereto. The invention also provides a low molecular weight fraction of a casein hydrolysate of the invention, and its use as a food ingredient, typically in foods such as infant formula, geriatric food products, sports and nutritional foods and supplements, and food products for immunocompromised individuals. The low molecular weight fraction of a casein hydrolysate of the invention is shown below to have highly reduced antigenicity compared to sodium caseinate, and sodium caseinate hydrolysates. The molecular weight profile of the peptides in the low molecular weight fraction of the casein hydrolysate of the invention is shown in Fig. 9 and the Sequence Listing appended hereto.

The term "low molecular weight fraction" should be understood as meaning that the hydrolysate is substantially free of peptides having a MW greater than 7kDa, 6kDa, and ideally 5kDa. Generally, it is obtained by separation of a hydrolysate of the invention to remove peptides or proteins have a MW greater than a cut-off value, ie 5KD, and retaining the permeate. In one embodiment, the low molecular weight fraction is substantially free of peptides having a MW greater than 4kDa, 3.5kD and ideally 3kDa Methods of removing peptide and protein fractions having a MW greater than a specified cut-off value are well known in the art, for example ultrafiltration and dialysis.

The term "substantially free of peptides having a molecular weight of greater than XkDa" should be understood to mean that the hydrolysate contains less than 2% (by weight of total peptide in hydrolysate) of peptides or proteins having a MW greater than XkDa, and ideally less than 1.5% or 1% (w/w) of peptides having a MW of greater than XkDa. In this regard, it should be noted that the hydrolysate of the invention, and fractions thereof, may contain trace amounts of peptides derived from other milk proteins, for example lactoglobulins.

In this specification, the term "controlled hydrolysis" should be understood to mean that the casein substrate is hydrolysed to a degree of hydrolysis of up to 15% (15%DH), preferably up to 14%DH, 13%DH or 12%DH. Ideally, the term should be understood to mean a degree of hydrolysis of at least 5%DH, 6%DH, 7%DH, 8%DH, 9%DH or 10%DH. In one embodiment, the term should be understood to mean a %DH of from 5%DH to 15%DH, 6%DH to 14%DH, 7%DH to 13%DH, or 8%DH to 12%DH.

%DH is measured using the technique described in below.

The term "*aspergillus*-derived proteolytic preparation" should be understood to mean a protease, or protease formulation, derived from *aspergillus* fungus, for example *aspergillus* oryzae. Examples of suitable protease activities include *aspergillus*-derived serine proteases and *aspergillus*-derived calcium-dependent proteases. In one embodiment, the proteolytic preparation employs more than one protease, for example at least two, three, four or five proteases. In a preferred embodiment, an *aspergillus*-derived serine protease and an *aspergillus*-derived calcium-dependent protease are employed. Ideally, the *aspergillus*-derived protease comprises a protease preparation of the FLAVORPRO-WHEY™ family, for example FLAVORPRO-WHEY™ 750-P or FLAVORPRO-WHEY™ 192-P.

The casein substrate typically comprises a casein salt, examples of which will be well known to those skilled in the art and include sodium caseinate. In a preferred embodiment, the casein substrate is a milk casein, ideally a bovine milk casein. Typically, the casein substrate (and/or the casein hydrolysate) is substantially free (i.e. less than 5%, 4%, 3%, 2%, 1%, 0.5%, 0.2%, 0.1% (w/w) of other milk proteins, for example whey proteins. The casein substrate employed for the hydrolysis typically has a concentration of 5-15%, 8-12%, and ideally about 9-11% (w/v) Typically, the ratio of protease to casein substrate (w.w) is from 0.1 to 1.0%, preferably 0.2 to 0.8%, more preferably 0.4 to 0.6%, and ideally at about 0.6%. (0.625-0.658 % (w:w) - see generation of hydrolysates section). Typically, the protease employed has an activity of >55 Casein Protease units/gram.

Suitably, hydrolysis of the casein substrate with protease is carried out at a temperature of from 45°C to 55°C, preferably from 49°C to 51°C, and ideally at about 50°C. Typically, hydrolysis of the casein substrate with protease is carried out at a pH of from 5 to 9, suitably at a pH of from 5.5 to 8, preferably at a pH of from 6 to 8, and ideally at a pH of about 7.

In a preferred embodiment, the casein hydrolysate of the invention has a peptide profile substantially similar to that shown in Fig. 10. In this regard, the term "substantially similar" should be understood to mean comprising at least 80%, 85%, 90%, 91%, 92%, 93%, 94%, 95%, 96%, 97%, 98%, 99% or 100% of the casein peptides of Figure 10 - SEQUENCE ID NO's 46 to 83 (for example. comprising 35 of the 38 peptides listed in Fig. 10)

In a preferred embodiment, the low molecular weight fraction of the casein hydrolysate of the invention has a peptide profile substantially similar to that shown in Fig. 9. In this regard, the term "substantially similar" should be understood to mean comprising at least 80%, 85%, 90%, 91%, 92%, 93%, 94%, 95%, 96%, 97%, 98%, 99% or 100% of the casein peptides of Figure 9 - SEQUENCE ID NO's 1 to 45 and 55 (for example. comprising 40 of the 46 casein-derived peptides listed in Fig. 9).

Thus, in a preferred embodiment, the invention provides a low molecular weight fraction of a casein hydrolysate fraction formed by hydrolyzing a milk (typically bovine milk) casein substrate to a degree of hydrolysis of 5%DH to 15%DH, 6%DH to 14%DH, 7%DH to 13%DH, or 8%DH to 12%DH, by a mixture of an *aspergillus*-derived serine protease and an *aspergillus*-derived calcium dependent protease, preferably a FlavorPro Whey™ protease preparation, in which the hydrolysate is substantially free of peptides having a MW of greater than 5kDa.

In another embodiment, the invention provides a casein hydrolysate formed by hydrolyzing a casein substrate to a degree of hydrolysis of from 8%DH to 14%DH by a mixture of an aspergillus-derived serine protease and an aspergillus-derived calcium dependent protease, preferably a FlavorPro Whey™ protease preparation, in which the hydrolysate is substantially free of peptides having a MW of greater than 7kDa, 6kDa, or ideally 5kDa.

The invention also relates to a casein hydrolysate of the invention in a dehydrated form, for example a powder. Methods of dehydrating the casein hydrolysate of the invention will be well known to those skilled in the art, and include fluidized bed drying, drum drying and spray drying.

Preferably, the casein hydrolysate of the invention comprises of peptides spread over the following distribution, as a function of their molecular mass:

>10kD - 0 to 30%
5-10kD - 0.1 to 8%
<5kD - 60 to 99.7%.

Preferably, the low molecular weight fraction of the casein hydrolysate of the invention comprises of peptides spread over the following distribution, as a function of their molecular mass:

>10kD-0 to 1%
5-10kD - 0.1 to 1%
<5kD - 98 to 99.9%.

Experimental Methods and Materials

**Hydrolysate generation-lab scale**

Sodium caseinate (NaCN, 85.92% w/w protein) was provided by Arrabawn Co-op Society Ltd., Tipperary, Ireland. NaCN solutions (4.8 L) of 9.3 % (w/v) were prepared the day before hydrolysis experiments by adding the appropriate quantity of NaCN powder to 4.5 L of distilled water. The protein was allowed to hydrate at 50 °C with gentle stirring in a sealed reaction vessel using a Heidolph overhead stirrer for 2 to 3 hours before being stored at 4 °C overnight. The following day, these solutions were subdivided into different aliquots for the generation of hydrolysates at different DH values.

**Table 1 Preparation of NaCN hydrolysates using FlavorPro Whey**

| Sample DH (%) | *E:S ratio, % |
|---|---|
| 0 (Intact NaCN) | - |
| 0.40 | 0.031 |
| 1.35 | 0.313 |
| 2.50 | 0.625 |
| 6.46 | " |
| 7.54 | " |
| 9.66 | " |
| 10.76 | " |
| * %(w:w) = %of enzyme powder/weight of NaCN protein present | |

Different E:S ratios were used to generate casein hydrolysates having different DH values. An E:S ratio of 0.625 % (w:w) was used to generate the FlavorPro Whey 10.76 % DH hydrolysate (Table 1).

Upon addition of the commercial proteolytic preparation, the pH of the protein solution was kept constant at pH 7 using a Titrino 718 pH stat. The temperature of the hydrolyzing NaCN solutions was maintained at 50 °C. Proteolytic activity was inactivated by bringing the temperature of the hydrolysate samples to 80°C and maintaining the samples at this temperature for a further 20 mins.

**Hydrolysate generation-semi-pilot scale**

A 300 L solution of 8.85 % (w/v) NaCN at pH 7 and 50 °C was prepared by mixing with a Silverson high shear mixer in a sealed water jacketed 300 L tank. FlavorPro Whey (174.70 g) was added to the stirred solution at an enzyme powder:substrate (E:S) ratio of 0.658 % (w:w).

The solution was incubated for 4 h at 50 °C in the 300 L tank and its pH was maintained at pH 7.0 with the addition of NaOH. The proteolytic activity was inactivated by bringing the temperature of the 300 L solution to 80 °C and maintaining it at that temperature for 20 mins. An aliquot of the hydrolysate was spray dried using a Niro Minor spray dryer. The remaining crude hydrolysate solution was then cooled to 5 °C and stored overnight. The following morning, the solution was reheated to 50 °C and following clarification was ultrafiltered through a membrane having an effective molecular mass cut off of 5 kDa mounted on a DSS MemProc membrane filtration unit. The resulting permeate and retentate were then spray dried using a Niro Minor spray dryer.

**Quantification of Degree of hydrolysis using TNBS**

Aliquots of the NaCN hydrolysates were diluted to ~0.09 % or 0.05 % (w/v) protein with 1 % (w/v) SDS and heated for 20 min at 80 °C to fully disperse the hydrolyzed protein. The Degree of hydrolysis (DH, %) of the NaCN hydrolysates was then determined using the TNBS method of Adler-Nissen (1979), as described by Spellman et al., (2003).

**Residual Antigenicity**

This was quantified using a sandwich format casein ELISA protocol employing polyclonal IgG (purified from serum) raised in rabbit against unhydrolyzed sodium caseinate. The purified IgG was coated onto standard medium bind 96 well microtitre plates, and was also conjugated to horse radish peroxidase (HRP) to make the "antibody conjugate". A calibration curve was constructed using the sodium caseinate used to immunize the rabbits for the calibration standard. The calibration curve range was between 10 and 10,000 ng/m/L which was plotted as 1 to 4 Log ng/mL. Aliquots of the NaCN hydrolysate samples were analysed for residual antigenicity to intact casein using the polyclonal antisera raised in rabbits. Samples were prepared at concentrations of 10, 1, 0.1 and 0.01mg/m (7, 6, 5 and 4 Log ng/mL) based on their protein content. Plots of ELISA response ($A_{450}/_{600}$) versus Log protein concentration were created to determine the relative reduction in residual antigenicity to intact casein (expressed as Log reduction and relative decrease).

**Gel Permeation Chromatography and Molecular Mass Distributions**

Gel permeation HPLC (GP-HPLC) was performed using the Waters HPLC system as essentially described by Spellman et al. (2005). A calibration curve was prepared from the average retention times of standard proteins and peptides (Smyth & FitzGerald, 1998). Molecular Mass Distributions were generated by integrating the GP-HPLC chromatograms using Breeze™ version 3.30 software, using the retention times corresponding to 5000 Da and 10000 Da to divide the chromatogram into 3 sections: 0-5000 Da, 5000-10000 Da and >10000 Da. The integrated area of each section was expressed as a percentage of the integrated area of the entire chromatogram obtained at 214 nm.

**Bitterness evaluation**

Hydrolysate samples, made up in non sparkling mineral water (Ballygowan) at a protein equivalent of 4.5 g/L, were randomly presented in triplicate to a 10-member sensory panel which had been trained to detect and quantify bitterness using caffeine solutions that were also made up in non-sparkling water. Panelists were trained to assign bitterness scores to unknown solutions based on a 0-100 % scale, where a 100 % bitter solution was taken to have a bitterness value equivalent to 1 g caffeine/L. Non sparkling mineral water was used as the 0 bitterness standard. At each sitting, panelists were presented with solutions of 0.00, 0.25, 0.50, 0.75 and 1.00 g caffeine/1, which had been labeled as 0, 25, 50, 75 and 100 and the test hydrolysates. The panelists were first required to taste the 0, 25, 50, 75 and 100 solutions in order to familiarise themselves with their taste intensities. They were then required to taste hydrolysates and based on their evaluations of the taste intensities of the 0, 25, 50, 75 and 100 solutions and then rank the taste intensities of the test hydrolysates out of 100. Between tasting each of the bitterness standards and hydrolysates, panelists were asked to eat a piece of non-salted cracker and to rinse their mouths thoroughly with non-sparkling mineral water (Spellman et al. (2005)). Results were expressed as the mean bitterness score $\pm$ standard error of the mean (SEM).

**Determination of Nitrogen solubility index**

This was carried out essentially according to Flanagan and FitzGerald (2002). Nitrogen was determined on the supernatants of 4 g 100 g$^{-1}$ aqueous protein solutions following centrifugation (1620 x g; 15 min), using a modification of the macro-Kjeldahl method (IDF, 1993). Kjeldahl catalyst tablets were used instead of potassium and copper sulphate, and the end-point of the titration step was reached at pH 4.6. All nitrogen solubility analyses were carried out induplicate.

**Mass spectral characterization of test samples**

The MS and tandem MS experiments were run on a MicrOTOF II mass spectrometer (Bruker Daltonics, Bremen, Germany) equipped with an electrospray ion source, controlled by the MicrOTOF Control software(version 2.3, Bruker Daltonics). The data were acquired over a mass/charge (m/z) range of 300-2500. A full scan MS spectrum was acquired, followed by tandem mass spectra using collision-induced dissociation (CID) of the five most intense precursor ions present in the MS scan. Electrospray conditions were as follows: capillary temperature, 130C; Capillary voltage, -1500V; Dry Gas flow, 6.0 L min$^{-1}$. Data analysis software (version 4.0, Bruker Daltonics), BioTool (version 3.1, Bruker Daltonics) and MASCOT (Perkins et al., 1999) were used for data processing and evaluation of peptide sequencing.

**Clarity Determination**

Clarity was determined at both room (20 °C) and storage temperature (4 °C) by measuring the absorbance at 660 nm of aqueous hydrolysate solutions using distilled water as blank essentially as described by Sin et al., (2006)

**Heat Stability Determination**

The pH of aliquots of aqueous NaCN hydrolysates (2.0% (w/v) protein equivalent) were adjusted to between 2.0 and 8.0, using 0.1 M and 1 M HCl or NaOH. Samples were then allowed to equilibrate for at least 1 h at room temperature. The pH of the samples was then re-measured and re-adjusted, if necessary, prior to heat stability analysis. Immediately after final pH adjustment, samples (2 ml) were placed in glass tubes (10 mm i.d._ 120 mm, AGB Scientific, Dublin, Ireland), sealed with silicone bungs, immersed in an oil bath thermostatically controlled at 140°C (Elbanton BV, Kerkdriel,The Netherlands), with continuous rocking at motor speed setting 3. The heat coagulation time (HCT) was taken as the length of time in min that elapsed between placing the sample in the oil bath and the onset of coagulation (Ryan et al., 2004). These analyses were performed in triplicate.

References

Adler-Nissen J (1979). Determination of the degree of hydrolysis of food protein hydrolysates by trinitrobenzenesulfonic acid. Journal ofAgricultural and Food Chemistry 27: 1256-1262

Flanagan, J. and FitzGerald, R. J. (2002). Physicochemical and nitrogen solubility properties of Bacillus proteinase hydrolysates of sodium caseinate incubated with transglutaminase pre- and post-hydrolysis. J. Agric. Food Chem. 50(19): 5429-5436.

IDF. (1993). Block Digestion (Macro) Method. International Standard 20B, Part 2. Brussels, Belgium: International Dairy Federation

Perkins, D. N. , Creasey, D. M. and Cottrell, J. S. (1999). Probability-based protein identification by searching sequence databases using mass spectrometry data. Electrophoresis, 20, 3551-3567.

Ryan, M., McEvoy, E., Duignan, S., Crowley, C., Fenelon, M. O' Callaghan, D.M. and FitzGerald, R.J. (2008). Thermal stability of soy protein isolate and hydrolysate ingredients. Food Chem. 108:503-510.

Sin, H.N., Yusof, S., Hamid, N. Sheikh Abdul and Rahman, R. Abd. (2006). Optimization of enzymatic clarification of sapodilla juice using response surface methodology. Journal of Food Engineering 73: 313-319.

Smyth M & FitzGerald R.J. 1998. Relationship between some characteristics of WPC hydrolysates and the enzyme complement in commercially available proteinase preparations. Int. Dairy Journal 8: 819-827

Spellman, D. O'Cuinn, G. and FitzGerald, R. J. (2005) Physicochemical and sensory characteristics of whey protein hydrolysates generated at different total solids levels. J. Dairy Res. 72: 138-143.

Spellman D, McEvoy E, O'Cuinn G & FitzGerald RJ (2003). Proteinase andexopeptidase hydrolysis of whey protein: Comparison of the TNBS,

OPA and pH stat methods for the quantification of degree of hydrolysis. International Dairy Journal 13: 447-453

## Claims

1. A casein hydrolysate formed by hydrolysis of a casein substrate by a *aspergillus*-derived proteolytic preparation to a degree of hydrolysis (%DH) of from 5%DH to 15%DH, the hydrolysate having a peptide profile that exhibits at least a 98% reduction in antigenicity compared to intact sodium caseinate and a mean bitterness score of less than 30% in which a bitterness score of 100% correlates with a 1g/L caffeine solution made up in non sparkling mineral water and a bitterness score of 0% correlates with non sparkling mineral water.

2. A casein hydrolysate of Claim 1 in which the degree of hydrolysis (%DH) is from 9%DH to 15%DH.

3. A casein hydrolysate of Claim 1 or 2 in which the *aspergillus*-derived proteolytic preparation is a FlavorPro-Whey™ proteolytic preparation.

4. A casein hydrolysate as claimed in any preceding Claim having a peptide profile that exhibits a mean bitterness score of less that 20%.

5. A low molecular weight fraction of a casein hydrolysate of any preceding Claim, which is substantially free of peptides or proteins having a molecular weight of greater than 5kD, the low molecular weight fraction having a peptide profile that exhibits a relative decrease in antigenicity compared to intact sodium caseinate of at least 100,000 and a mean bitterness score of less than 30%.

6. A low molecular weight fraction of Claim 5, **characterized in that** the peptide profile of the low molecular weight fraction is spread over the following distribution, as a function of their molecular mass:

> 10kD - 0 to 1%
5-10kD - 0.1 to 1%
<5kD - 98 to 99.9%.

7. A low molecular weight fraction of Claim 5 or 6, the low molecular weight fraction exhibiting a relative decrease in antigenicity compared to intact sodium caseinate of at least 800,000.

8. A low molecular weight fraction of Claim 5, 6 or 7, having a peptide profile that exhibits a mean bitterness score of less that 20%.

9. An infant formula comprising a casein hydrolysate of any of Claims 1 to 4 or a low molecular weight fraction of Claim 5 to 8.

10. A casein hydrolysate of any of Claims 1 to 4 for use as a food ingredient, a foodstuff or beverage for infants, geriatrics, or immunocompromised individuals, or a general or a sports nutrition supplement.

11. A method for producing a casein hydrolysate, according to claim 1, the method comprising the steps of hydrolyzing a casein substrate to a degree of hydrolysis of from 5%DH to 15%DH using an *aspergillus*-derived proteolytic preparation.

12. A method as claimed in Claim 11 in which the *aspergillus*-derived proteolytic preparation is a FlavorPro Whey™ proteolytic preparation.

13. A method as claimed in Claim 11 or 12 in which the casein substrate is hydrolysed to a degree of hydrolysis (%DH) is from 9%DH to 15%DH.

14. A method for producing a low molecular weight fraction of a casein hydrolysate, according to claim 1, the method comprising the steps of hydrolyzing a casein substrate to a degree of hydrolysis of from 5%DH to 15%DH using a *aspergillus*-derived proteolytic preparation and fractionating the hydrolysate using separation means to provide a low molecular weight fraction which is substantially free of peptides having a MW of greater than 5kDa.

15. A method as claimed in Claim 14 in which the *aspergillus*-derived proteolytic preparation is a FlavorPro Whey™ proteolytic preparation; and/or in which the casein substrate is hydrolysed to a degree of hydrolysis (%DH) from 9%DH to 15%DH.

**Patentansprüche**

1. Caseinhydrolysat, das durch Hydrolyse eines Caseinsubstrats durch ein von *Aspergillus* stammendes proteolytisches Präparat bis zu einem Hydrolysegrad (%-DH) von 5 %-DH bis 15 %-DH gebildet wird, wobei das Hydrolysat ein Peptidprofil aufweist, das mindestens ein Reduzierung der Antigenität von 98 %, im Vergleich zum intakten Natriumcaseinat, und einen mittleren Bitterkeitswert von weniger als 30 % zeigt, in dem ein Bitterkeitswert von 100 % einer 1 g/l Koffeinlösung entspricht, die in stillem Mineralwasser hergestellt wird, und ein Bitterkeitswert von 0 % stillem Mineralwasser entspricht.

2. Caseinhydrolysat nach Anspruch 1, in dem der Hydrolysegrad (%-DH) von 9 %-DH bis 15 %-DH beträgt.

3. Caseinhydrolysat nach Anspruch 1 oder 2, in dem das von *Aspergillus* stammende proteolytische Präparat ein proteolytisches FlavorPro-Whey™ Präparat ist.

4. Caseinhydrolysat nach einem vorstehenden Anspruch, das ein Peptidprofil aufweist, das einen mittleren Bitterkeitswert von weniger als 20 % zeigt.

5. Niedermolekulare Gewichtsfraktion eines Caseinhydrolysats nach einem vorstehenden Anspruch, die im Wesentlichen frei von Peptiden oder Proteinen ist, die ein Molekulargewicht von größer als 5 kD aufweisen, wobei die niedermolekulare Gewichtsfraktion ein Peptidprofil aufweist, das eine relative Abnahme der Antigenität, im Vergleich zum intakten Natriumcaseinat, von mindestens 100 000 und einen mittleren Bitterkeitswert von weniger als 30 % zeigt.

6. Niedermolekulare Gewichtsfraktion nach Anspruch 5, **dadurch gekennzeichnet, dass** sich das Peptidprofil der niedermolekularen Gewichtsfraktion auf die folgende Aufteilung, als Funktion ihrer Molekularmasse, verteilt:

>10 kD - 0 bis 1 %
5-10 kD - 0,1 bis 1 %
<5 kD - 98 bis 99,0 %.

7. Niedermolekulare Gewichtsfraktion nach Anspruch 5 oder 6, wobei die niedermolekulare Gewichtsfraktion eine relative Abnahme der Antigenität, im Vergleich zum intakten Natriumcaseinat, von mindestens 800 000 zeigt.

8. Niedermolekulare Gewichtsfraktion nach Anspruch 5, 6 oder 7, die ein Peptidprofil aufweist, das einen mittleren Bitterkeitswert von weniger als 20 % zeigt.

9. Säuglingsnahrung, die ein Caseinhydrolysat nach einem der Ansprüche 1 bis 4 oder eine niedermolekulare Gewichtsfraktion nach Ansprüchen 5 bis 8 umfasst.

10. Caseinhydrolysat nach einem der Ansprüche 1 bis 4 für die Verwendung als Nahrungsmittelbestandteil, als Nahrungsmittel oder Getränk für Säuglinge, geriatrische Patienten oder immunkompromittierte Individuen oder als allgemeine Nahrungsergänzung oder Nahrungsergänzung für Sportler.

11. Verfahren zur Herstellung eines Caseinhydrolysats nach Anspruch 1, wobei das Verfahren die folgenden Schritte umfasst:

Hydrolysieren eines Caseinsubstrats bis zu einem Hydroysegrad von 5 %-DH bis 15 %-DH unter Verwendung von einem von *Aspergillus* stammenden proteolytischen Präparat.

12. Verfahren nach Anspruch 11, in dem das von *Aspergillus* stammende proteolytische Präparat ein proteolytisches FlavorPro-Whey™ Präparat ist.

13. Verfahren nach Anspruch 11 oder 12, in dem das Caseinsubstrat bis zu einem Hydrolysegrad (%-DH) von 9 %-DH bis 15 %-DH hydrolysiert wird.

14. Verfahren zur Herstellung einer niedermolekularen Gewichtsfraktion eines Caseinhydrolysats nach Anspruch 1, wobei das Verfahren die folgenden Schritte umfasst:

Hydrolysieren eines Caseinsubstrats bis zu einem Hydroysegrad von 5 %-DH bis 15 %-DH unter Verwendung von einem von *Aspergillus* stammenden proteolytischen Präparat und Fraktionieren des Hydrolysats unter Verwendung von Trennungsmitteln, um eine niedermolekulare Gewichtsfraktion bereitzustellen, die im Wesentlichen frei von Peptiden ist, die ein MW von größer als 5 kD aufweisen.

15. Verfahren nach Anspruch 14, in dem das von *Aspergillus* stammende proteolytische Präparat ein proteolytisches FlavorPro-Whey™ Präparat ist; und/oder in dem das Caseinsubstrat bis zu einem Hydrolysegrad (%-DH) von 9 %-DH bis 15 %-DH hydrolysiert wird.

**Revendications**

1. Hydrolysat de caséine formé par l'hydrolyse d'un substrat de caséine par une préparation protéolytique dérivée d'*Aspergillus* jusqu'à un degré d'hydrolyse (% de DH) allant de 5% de DH à 15% de DH, l'hydrolysat ayant un profil peptidique qui présente une réduction d'au moins 98% de l'antigénicité par rapport à un caséinate de sodium intact et un score d'amertume moyen inférieur à 30% où un score d'amertume de 100% est corrélé avec une solution de caféine de 1g/l préparée dans de l'eau minérale non gazeuse et un score d'amertume de 0% est corrélé avec de l'eau minérale non gazeuse.

2. Hydrolysat de caséine selon la revendication 1, dans lequel le degré d'hydrolyse (% de DH) va de 9% de DH à 15% de DH.

3. Hydrolysat de caséine selon la revendication 1 ou 2, dans lequel la préparation protéolytique dérivée d'*Aspergillus* est une préparation protéolytique FlavorPro-Whey ™.

4. Hydrolysat de caséine selon l'une quelconque des revendications précédentes, ayant un profil peptidique qui présente un score d'amertume moyen inférieur à 20%.

5. Fraction de bas poids moléculaire d'un hydrolysat de caséine selon l'une quelconque des revendications précédentes, qui est sensiblement dépourvu de peptides ou de protéines ayant un poids moléculaire supérieur à 5 kDa, la fraction de bas poids moléculaire ayant un profil peptidique qui présente une réduction relative d'antigénicité par rapport à un caséinate de sodium intact d'au moins 100 000 et un score d'amertume moyen inférieur à 30%.

6. Fraction de bas poids moléculaire selon la revendication 5, **caractérisé en ce que** le profil peptidique de la fraction de bas poids moléculaire est réparti selon la distribution suivante, en fonction de leur masse moléculaire :

   >10kDa - 0 à 1%
   5-10 kDa-0,1 à 1%
   < 5kDa - 98 à 99,9%.

7. Fraction de bas poids moléculaire selon la revendication 5 ou 6, la fraction de bas poids moléculaire présentant une réduction relative d'antigénicité par rapport à un caséinate de sodium intact d'au moins 800 000.

8. Fraction de bas poids moléculaire selon la revendication 5, 6 ou 7, ayant un profil peptidique qui présente un score d'amertume moyen inférieur à 20%.

9. Préparation pour nourrissons comprenant un hydrolysat de caséine selon l'une quelconque des revendications 1 à 4 ou une fraction de bas poids moléculaire selon les revendications 5 à 8.

10. Hydrolysat de caséine selon l'une quelconque des revendications 1 à 4, pour une utilisation comme ingrédient alimentaire, produit alimentaire ou boisson pour nourrissons, personnes âgées ou individus immunodéprimés, ou comme complément de nutrition pour sportifs ou générale.

11. Méthode de production d'un hydrolysat de caséine selon la revendication 1, la méthode comprenant les étapes consistant à hydrolyser un substrat de caséine jusqu'à un degré d'hydrolyse allant de 5% de DH à 15% de DH par une préparation protéolytique dérivée d'*Aspergillus*.

12. Méthode selon la revendication 11, dans laquelle la préparation protéolytique dérivée d'*Aspergillus* est une préparation protéolytique FlavorPro-Whey ™.

13. Méthode selon la revendication 11 ou 12, dans laquelle le substrat de caséine est hydrolysé jusqu'à un degré d'hydrolyse (% de DH) allant de 9% de DH à 15% de DH.

14. Méthode de production d'une fraction de bas poids moléculaire d'un hydrolysat de caséine selon la revendication 1, la méthode comprenant les étapes consistant à hydrolyser un substrat de caséine jusqu'à un degré d'hydrolyse allant de 5% de DH à 15% de DH par une préparation protéolytique dérivée *d'Aspergillus* et le fractionnement de l'hydrolysat en utilisant un moyen de séparation afin de fournir une fraction de bas poids moléculaire qui est sensiblement dépourvue de peptides ayant un PM supérieur à 5 kDa.

**15.** Méthode selon la revendication 14, dans laquelle la préparation protéolytique dérivée *d'Aspergillus* est une préparation protéolytique FlavorPro-Whey ™ ; et/ou dans laquelle le substrat de caséine est hydrolysé jusqu'à un degré d'hydrolyse (% de DH) allant de 9% de DH à 15% de DH.

**FIGURE 1**

| Degree of Hydrolysis (DH), % | Log reduction in antigenicity | Relative decrease in sample antigenicity compared to sodium caseinate antigenicity |
|---|---|---|
| 0.40 | 0 | 0 |
| 1.35 | 0 | 0 |
| 2.5 | 0 | 0 |
| 6.46 | 1.75 | 56.23 |
| 7.54 | 1.75 | 56.23 |
| 9.66 | 2.7 | 501.19 |
| 10.76 | 2.9 | 794.33 |

**FIGURE 2**

| FlavorPro Whey semi-pilot scale NaCN hydrolysate | Log reduction in antigenicity | Relative decrease in sample antigenicity compared to intact sodium caseinate antigenicity |
|---|---|---|
| Crude hydrolysate | 2.8 | 631 |
| 5 kD permeate fraction | 6 | 1000000 |

**FIGURE 3**

| Sample | Molecular Mass Distribution (% Area) | | |
|---|---|---|---|
| | >10 kDa | 5-10 kDa | <5 kDa |
| Intact NaCN | 97.73 | 1.45 | 0.72 |
| Crude hydrolysate | 24.48 | 4.59 | 70.93 |
| 5 kD permeate | 0.00 | 0.53 | 99.47 |

FIGURE 4

FIGURE 5

**FIGURE 6**

**FIGURE 7**

**FIGURE 8**

(A)

Clarity of #37 semi-pilot NaCN hydrolysate at 4 °C

(B)

Clarity of #37 semi-pilot NaCN hydrolysate at 20 °C

FIGURE 10

| Parent Protein | Sequence position | Amino Acid Sequence (SEQ ID Number) | Measured Molecular Mass (Daltons) | Calculated Molecular Mass (Daltons) |
|---|---|---|---|---|
| Alpha-S1-casein | 26 - 32 | APFPEVF (46) | 805.39 | 805.401 |
| | 129 - 139 | AQQKEPMIGVN (47) | 1213.588 | 1213.612 |
| | 128 - 139 | HAQQKEPMIGVN (48) | 1350.647 | 1350.671 |
| | 81 - 91 | IQKEDVPSERY (49) | 1362.654 | 1362.678 |
| | 8 - 19 | HQGLPQEVLNEN (50) | 1376.644 | 1376.668 |
| | 129 - 141 | AQQKEPMIGVNQE (51) | 1470.688 | 1470.714 |
| | 8 - 20 | HQGLPQEVLNENL (52) | 1489.734 | 1489.752 |
| | 26 - 39 | APFPEVFGKEKVNE (53) | 1589.784 | 1589.809 |
| | 7 - 20 | KHQGLPQEVLNENL (54) | 1617.827 | 1617.847 |
| | 106 - 119 | VPQLEIVPNSAEER (55) | 1659.767 | 1659.787 |
| | 6 - 20 | IKHQGLPQEVLNENL (56) | 1730.906 | 1730.932 |
| | 105 - 119 | KVPQLEIVPNSAEER (57) | 1787.851 | 1787.882 |
| | 180 - 197 | SDIPNPIGSENSEKTTMP (58) | 1915.854 | 1915.883 |
| | 104 - 119 | YKVPQLEIVPNSAEER (59) | 1950.918 | 1950.945 |
| | 104 - 119 | YKVPQLEIVPNSAEER (59) | 1950.92 | 1950.945 |
| | 106 - 122 | VPQLEIVPNSAEERLHS (60) | 1996.935 | 1996.962 |
| | 179 - 197 | FSDIPNPIGSENSEKTTMP (61) | 2062.924 | 2062.952 |

EP 2 675 299 B1

| Parent Protein | Sequence position | Amino Acid Sequence (SEQ ID Number) | Measured Molecular Mass (Daltons) | Calculated Molecular Mass (Daltons) |
|---|---|---|---|---|
| | 103 - 119 | KYKVPQLEIVPNSAEER (62) | 2079.001 | 2079.04 |
| | 102 - 119 | KKYKVPQLEIVPNSAEER (63) | 2207.097 | 2207.135 |
| | 1 - 20 | RPKHPIKHQGLPQEVLNENL (64) | 2346.245 | 2346.292 |
| Alpha-S2-casein | 175 - 179 | ALPQY (65) | 590.3 | 590.306 |
| | 116 - 122 | AVPITPT (66) | 697.389 | 697.401 |
| | 102 - 115 | GPIVLNPWDQVKRN (67) | 1634.857 | 1634.889 |
| | 101 - 114 | QGPIVLNPWDQVKR (68) | 1648.88 | 1648.905 |
| | 101 - 115 | QGPIVLNPWDQVKRN (69) | 1762.921 | 1762.948 |
| | 100 - 114 | YQGPIVLNPWDQVKR (70) | 1811.944 | 1811.968 |
| | 100 - 115 | YQGPIVLNPWDQVKRN (71) | 1925.985 | 1926.011 |
| Beta-casein | 126 - 132 | TLTDVEN (72) | 790.36 | 790.371 |
| | 177 - 186 | AVPYPQRDMP (73) | 1172.544 | 1172.565 |
| | 107 - 118 | KEMPFPKYPVEP (74) | 1460.712 | 1460.737 |
| | 177 - 189 | AVPYPQRDMPIQA (75) | 1484.719 | 1484.745 |
| | 106 - 118 | HKEMPFPKYPVEP (76) | 1597.781 | 1597.796 |
| | 177 - 190 | AVPYPQRDMPIQAF (77) | 1631.794 | 1631.813 |

EP 2 675 299 B1

| 194 - 209 | QEPVLGPVRGPFPIIV (78) | 1716.965 | 1716.993 |
|---|---|---|---|
| 193 - 209 | YQEPVLGPVRGPFPIIV (79) | 1880.026 | 1880.056 |
| 28 - 44 | KKIEKFQSEEQQQTEDE (80) | 2202.933 | 2202.968 |
| 106 - 124 | HKEMPFPKYPVEPFTESQS (81) | 2277.043 | 2277.078 |
| 28 - 46 | KKIEKFQSEEQQQTEDELQ (82) | 2444.068 | 2444.111 |
| 166 - 189 | SQSKVLPVPQKAVPYPQRDMPIQA (83) | 2676.398 | 2676.442 |

FIGURE 9

| Parent Protein | Sequence position | Amino Acid Sequence (SEQ ID Number) | Measured Molecular Mass (Daltons) | Calculated Molecular Mass (Daltons) |
|---|---|---|---|---|
| Alpha-S1-casein | 92 - 98 | LGYLEQL (1) | 834.4612 | 834.4487 |
| | 156 - 164 | LDAYPSGAW (2) | 978.4615 | 978.4447 |
| | 26 - 34 | APFPEVFGK (3) | 990.531 | 990.5175 |
| | 111 - 119 | IVPNSAEER (4) | 1093.4984 | 1093.4805 |
| | 146 - 153 | YPELFRQF (5) | 1098.5646 | 1098.5498 |
| | 109 - 119 | LEIVPNSAEER (6) | 1335.6256 | 1335.6071 |
| | 8 - 19 | HQGLPQEVLNEN (7) | 1376.686 | 1376.6684 |
| | 8 - 20 | HQGLPQEVLNENL (8) | 1489.7782 | 1489.7525 |
| | 132 - 144 | KEPMIGVNQELAY (9) | 1490.7616 | 1490.7439 |
| | 80 - 91 | HIQKEDVPSERY (10) | 1499.7544 | 1499.7368 |
| | 132 - 144 | KEP<u>M</u>IGVNQELAY (11) | 1506.7586 | 1506.7388 |
| | 7 - 20 | KHQGLPQEVLNENL (12) | 1617.8632 | 1617.8474 |
| | 106 - 119 | VPQLEIVPN<u>S</u>AEER (55) | 1659.8094 | 1659.7869 |
| | 6 - 20 | IKHQGLPQEVLNENL (13) | 1730.9564 | 1730.9315 |

| Parent Protein | Sequence position | Amino Acid Sequence (SEQ ID Number) | Measured Molecular Mass (Daltons) | Calculated Molecular Mass (Daltons) |
|---|---|---|---|---|
| Alpha-S1-casein | 105 - 119 | KVPQLEIVPNSAEER (14) | 1787.9054 | 1787.8818 |
| | 106 - 121 | VPQLEIVPNSAEERLH (15) | 1909.9519 | 1909.9299 |
| | 180 - 197 | SDIPNPIGSENSEKTTMP (16) | 1931.9062 | 1931.8782 |
| | 104 - 119 | YKVPQLEIVPNSAEER (17) | 1950.973 | 1950.9452 |
| | 4 - 20 | HPIKHQGLPQEVLNENL (18) | 1965.0646 | 1965.0432 |
| | 106-122 | VPQLEIVPNSAEERLHS (19) | 1996.99 | 1996.9619 |
| | 105 - 121 | KVPQLEIVPNSAEERLH (20) | 2038.0525 | 2038.0248 |
| | 127 - 144 | IHAQQKEPMIGVNQELAY (21) | 2068.0552 | 2068.0411 |
| | 1 - 20 | RPKHPIKHQGLPQEVLNENL (22) | 2346.3139 | 2346.292 |

| Parent Protein | Sequence position | Amino Acid Sequence (SEQ ID Number) | Measured Molecular Mass (Daltons) | Calculated Molecular Mass (Daltons) |
|---|---|---|---|---|
| Alpha-S2-casein | 198 - 203 | TKVIPY (23) | 719.4256 | 719.4218 |
| | 174 - 179 | FALPQY (24) | 737.3833 | 737.3748 |
| | 83 - 88 | NEINQF (25) | 763.361 | 763.3501 |
| | 81 - 87 | ALNEINQ (26) | 800.4145 | 800.4028 |
| | 81 - 88 | ALNEINQF (27) | 947.4897 | 947.4712 |

| Parent Protein | Sequence position | Amino Acid Sequence (SEQ ID Number) | Measured Molecular Mass (Daltons) | Calculated Molecular Mass (Daltons) |
|---|---|---|---|---|
| | 154 - 163 | TEEEKNRLNF (28) | 1278.638 | 1278.6204 |
| | 101 - 114 | QGPIVLNPWDQVKR (29) | 1762.9639 | 1762.9478 |
| | 100 - 113 | YQGPIVLNPWDQVK (30) | 1811.9929 | 1811.9822 |
| | 100 - 114 | YQGPIVLNPWDQVKR (31) | 1926.0373 | 1926.0112 |
| Beta-casein | 203 - 209 | GPFPIIV (32) | 741.45 | 741.4425 |
| | 126 - 133 | TLTDVENL (33) | 903.473 | 903.4549 |
| | 199 - 209 | GPVRGPFPIIV (34) | 1150.6962 | 1150.6863 |
| | 128 - 139 | TDVENLHLPLPL (35) | 1359.754 | 1359.7398 |
| | 194 - 206 | QEPVLGPVRGPFP (36) | 1391.775 | 1391.7561 |
| | 193 - 206 | YQEPVLGPVRGPFP (37) | 1554.8454 | 1554.8195 |
| | 177 - 190 | AVPYPQRDMPIQAF (38) | 1631.8386 | 1631.813 |
| | 177 - 190 | AVPYPQRD_M_PIQAF (38) | 1647.8342 | 1647.8079 |
| | 194 - 209 | QEPVLGPVRGPFPIIV (39) | 1717.0194 | 1716.9927 |
| | 193 - 209 | YQEPVLGPVRGPFPIIV (40) | 1880.0844 | 1880.056 |
| | 106 - 124 | HKEMPFPKYPVEPFTESQS (41) | 2277.1099 | 2277.0776 |
| | 106 - 124 | HKE_M_PFPKYPVEPFTESQS (41) | 2293.1038 | 2293.0725 |
| | 33 - 52 | FQ_S_EEQQQTEDELQDKIHPF (42) | 2555.1241 | 2555.0853 |
| | 166 - 190 | SQSKVLPVPQKAVPYPQRDMPIQAF (43) | 2823.5385 | 2823.5105 |

| Parent Protein | Sequence position | Amino Acid Sequence (SEQ ID Number) | Measured Molecular Mass (Daltons) | Calculated Molecular Mass (Daltons) |
|---|---|---|---|---|
| Kappa-casein | 25 - 30 | YIPIQY  (44) | 795.4265 | 795.4167 |
| | 56 - 61 | LPYPYY  (45) | 814.4031 | 814.3901 |

| Parent Protein | Sequence position | Amino Acid Sequence | Measured Molecular Mass (Daltons) | Calculated Molecular Mass (Daltons) |
|---|---|---|---|---|
| Beta-lactoglobulin | 47 - 57 | KPTPEGDLEIL | 1210.6556 | 1210.6445 |
| | 47 - 58 | KPTPEGDLEILL | 1323.7428 | 1323.7286 |
| | 45 - 57 | ELKPTPEGDLEIL | 1452.7886 | 1452.7711 |
| | 43 - 57 | VEELKPTPEGDLEIL | 1680.9044 | 1680.8822 |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 2258208 A
- WO 9425580 A
- EP 0631731 A
- WO 2006130204 A
- EP 11154760 A

### Non-patent literature cited in the description

- **JINGLI et al.** *J. Dairy Res.,* 2007, vol. 74 (1
- **ADLER-NISSEN J.** Determination of the degree of hydrolysis of food protein hydrolysates by trinitrobenzenesulfonic acid. *Journal of Agricultural and Food Chemistry,* 1979, vol. 27, 1256-1262
- **FLANAGAN, J. ; FITZGERALD, R. J.** Physicochemical and nitrogen solubility properties of Bacillus proteinase hydrolysates of sodium caseinate incubated with transglutaminase pre- and post-hydrolysis. *J. Agric. Food Chem.,* 2002, vol. 50 (19), 5429-5436
- IDF. (1993). Block Digestion (Macro) Method. International Standard 20B. *International Dairy Federation*
- **PERKINS, D. N. ; CREASEY, D. M. ; COTTRELL, J. S.** Probability-based protein identification by searching sequence databases using mass spectrometry data. *Electrophoresis,* 1999, vol. 20, 3551-3567
- **RYAN, M. ; MCEVOY, E. ; DUIGNAN, S. ; CROWLEY, C. ; FENELON, M. ; O' CALLAGHAN, D.M. ; FITZGERALD, R.J.** Thermal stability of soy protein isolate and hydrolysate ingredients. *Food Chem.,* 2008, vol. 108, 503-510
- **SIN, H.N. ; YUSOF, S. ; HAMID, N. SHEIKH ABDUL ; RAHMAN, R. ABD.** Optimization of enzymatic clarification of sapodilla juice using response surface methodology. *Journal of Food Engineering,* 2006, vol. 73, 313-319
- **SMYTH M ; FITZGERALD R.J.** Relationship between some characteristics of WPC hydrolysates and the enzyme complement in commercially available proteinase preparations. *Int. Dairy Journal,* 1998, vol. 8, 819-827
- **SPELLMAN, D. ; O'CUINN, G. ; FITZGERALD, R. J.** Physicochemical and sensory characteristics of whey protein hydrolysates generated at different total solids levels. *J. Dairy Res.,* 2005, vol. 72, 138-143
- **SPELLMAN D ; MCEVOY E ; O'CUINN G ; FITZGERALD RJ.** *Proteinase andexopeptidase hydrolysis of whey protein: Comparison of the TNBS,* 2003
- OPA and pH stat methods for the quantification of degree of hydrolysis. *International Dairy Journal,* vol. 13, 447-453

SEQUENCE LISTING

<110> UNIVERSITY OF LIMERICK

<120> A CASEIN HYDROLYSATE

<130> P10643PC00

<150> EP 11154760.0
<151> 2011-02-17

<160> 83

<170> PatentIn version 3.5

<210> 1
<211> 7
<212> PRT
<213> Bos taurus

<220>
<221> MISC_FEATURE
<222> (1)..(7)
<223> Alpha S1 casein peptide

<400> 1

```
Leu Gly Tyr Leu Glu Gln Leu
1               5
```

<210> 2
<211> 9
<212> PRT
<213> Bos taurus

<220>
<221> MISC_FEATURE
<222> (1)..(9)
<223> Alpha S1 casein peptide

<400> 2

Leu Asp Ala Tyr Pro Ser Gly Ala Trp
1               5

<210> 3
<211> 9
<212> PRT
<213> Bos taurus

<220>
<221> MISC_FEATURE
<222> (1)..(9)
<223> Alpha S1 casein peptide

<400> 3

Ala Pro Phe Pro Glu Val Phe Gly Lys

                1               5

<210> 4
<211> 9
<212> PRT
<213> Bos taurus

<220>
<221> MISC_FEATURE
<222> (1)..(9)
<223> Alpha S1 casein peptide

<400> 4

Ile Val Pro Asn Ser Ala Glu Glu Arg
1               5

<210> 5
<211> 8
<212> PRT
<213> Bos taurus

<220>
<221> MISC_FEATURE
<222> (1)..(8)
<223> Alpha S1 casein peptide

<400> 5

Tyr Pro Glu Leu Phe Arg Gln Phe
1               5

<210> 6
<211> 11
<212> PRT
<213> Bos taurus

<220>
<221> MISC_FEATURE

<222> (1)..(10)
<223> Alpha S1 casein peptide

<400> 6

```
              Leu Glu Ile Val Pro Asn Ser Ala Glu Glu Arg
              1               5                   10
```

<210> 7
<211> 12
<212> PRT
<213> Bos taurus

<220>
<221> MISC_FEATURE
<222> (1)..(12)
<223> Alpha S1 casein peptide

<400> 7

```
              His Gln Gly Leu Pro Gln Glu Val Leu Asn Glu Asn
              1               5                   10
```

<210> 8
<211> 13
<212> PRT
<213> Bos taurus

<220>
<221> MISC_FEATURE
<222> (1)..(13)
<223> Alpha S1 casein peptide

<400> 8

```
              His Gln Gly Leu Pro Gln Glu Val Leu Asn Glu Asn Leu
              1               5                   10
```

<210> 9
<211> 13
<212> PRT
<213> Bos taurus

<220>
<221> MISC_FEATURE
<222> (1)..(13)
<223> Alpha S1 casein peptide

<400> 9

```
              Lys Glu Pro Met Ile Gly Val Asn Gln Glu Leu Ala Tyr
              1               5                   10
```

<210> 10
<211> 12
<212> PRT
<213> Bos taurus

```
<220>
<221> MISC_FEATURE
<222> (1)..(12)
<223> Alpha S1 casein peptide

<400> 10

            His Ile Gln Lys Glu Asp Val Pro Ser Glu Arg Tyr
            1                   5                   10

<210> 11
<211> 13
<212> PRT
<213> Bos taurus

<220>
<221> MISC_FEATURE
<222> (1)..(13)
<223> Alpha S1 casein peptide

<400> 11

            Lys Glu Pro Met Ile Gly Val Asn Gln Glu Leu Ala Tyr
            1                   5                   10

<210> 12
<211> 14
<212> PRT
<213> Bos taurus

<220>
<221> MISC_FEATURE
<222> (1)..(14)
<223> Alpha S1 casein peptide

<400> 12

            Lys His Gln Gly Leu Pro Gln Glu Val Leu Asn Glu Asn Leu
            1                   5                   10

<210> 13
<211> 15
<212> PRT
<213> Bos taurus

<220>
<221> INIT_MET
<222> (1)..(15)
<223> Alpha S1 casein peptide

<400> 13

            Ile Lys His Gln Gly Leu Pro Gln Glu Val Leu Asn Glu Asn Leu
            1                   5                   10                  15

<210> 14
<211> 15
<212> PRT
```

<213> Bos taurus

<220>
<221> MISC_FEATURE
<222> (1)..(15)
<223> Alpha S1 casein peptide

<400> 14

```
        Lys Val Pro Gln Leu Glu Ile Val Pro Asn Ser Ala Glu Glu Arg
        1               5                   10                  15
```

<210> 15
<211> 16
<212> PRT
<213> Bos taurus

<220>
<221> MISC_FEATURE
<222> (1)..(16)
<223> Alpha S1 casein peptide

<400> 15

```
        Val Pro Gln Leu Glu Ile Val Pro Asn Ser Ala Glu Glu Arg Leu His
        1               5                   10                  15
```

<210> 16
<211> 18
<212> PRT
<213> Bos taurus

<220>
<221> MISC_FEATURE
<222> (1)..(18)
<223> Alpha S1 casein peptide

<400> 16

```
        Ser Asp Ile Pro Asn Pro Ile Gly Ser Glu Asn Ser Glu Lys Thr Thr
        1               5                   10                  15


        Met Pro
```

<210> 17
<211> 16
<212> PRT
<213> Bos taurus

<220>
<221> MISC_FEATURE
<222> (1)..(16)
<223> Alpha S1 casein peptide

<400> 17

```
        Tyr Lys Val Pro Gln Leu Glu Ile Val Pro Asn Ser Ala Glu Glu Arg
        1               5               10              15
```

<210> 18
<211> 17
<212> PRT
<213> Bos taurus

<220>
<221> MISC_FEATURE
<222> (1)..(17)
<223> Alpha S1 casein peptide

<400> 18

```
        His Pro Ile Lys His Gln Gly Leu Pro Gln Glu Val Leu Asn Glu Asn
        1               5               10              15
```

        Leu

<210> 19
<211> 17
<212> PRT
<213> Bos taurus

<220>
<221> MISC_FEATURE
<222> (1)..(17)
<223> Alpha S1 casein peptide

<400> 19

```
        Val Pro Gln Leu Glu Ile Val Pro Asn Ser Ala Glu Glu Arg Leu His
        1               5               10              15
```

        Ser

<210> 20
<211> 17
<212> PRT
<213> Bos taurus

<220>
<221> MISC_FEATURE
<222> (1)..(17)
<223> Alpha S1 casein peptide

<400> 20

```
        Lys Val Pro Gln Leu Glu Ile Val Pro Asn Ser Ala Glu Glu Arg Leu
        1               5               10              15
```

        His

<210> 21

<211> 18
<212> PRT
<213> Bos taurus

<220>
<221> MISC_FEATURE
<222> (1)..(18)
<223> Alpha S1 casein peptide

<400> 21

```
Ile His Ala Gln Gln Lys Glu Pro Met Ile Gly Val Asn Gln Glu Leu
1               5               10              15

Ala Tyr
```

<210> 22
<211> 20
<212> PRT
<213> Bos taurus

<220>
<221> MISC_FEATURE
<222> (1)..(20)
<223> Alpha S1 casein peptide

<400> 22

```
Arg Pro Lys His Pro Ile Lys His Gln Gly Leu Pro Gln Glu Val Leu
1               5               10              15

Asn Glu Asn Leu
                20
```

<210> 23
<211> 6
<212> PRT
<213> Bos taurus

<220>
<221> MISC_FEATURE
<222> (1)..(6)
<223> Alpha S2 casein peptide

<400> 23

```
Thr Lys Val Ile Pro Tyr
1               5
```

<210> 24
<211> 6
<212> PRT
<213> Bos taurus

<220>
<221> MISC_FEATURE

<222> (1)..(6)
<223> Alpha S2 casein peptide

<400> 24

```
                                    Phe Ala Leu Pro Gln Tyr
                                    1               5
```

<210> 25
<211> 6
<212> PRT
<213> Bos taurus

<220>
<221> MISC_FEATURE
<222> (1)..(6)
<223> Alpha S2 casein peptide

<400> 25

```
                                    Asn Glu Ile Asn Gln Phe
                                    1               5
```

<210> 26
<211> 7
<212> PRT
<213> Bos taurus

<220>
<221> MISC_FEATURE
<222> (1)..(7)
<223> Alpha S2 casein peptide

<400> 26

```
                                Ala Leu Asn Glu Ile Asn Gln
                                1               5
```

<210> 27
<211> 8
<212> PRT
<213> Bos taurus

<220>
<221> MISC_FEATURE
<222> (1)..(8)
<223> Alpha S2 casein peptide

<400> 27

```
                            Ala Leu Asn Glu Ile Asn Gln Phe
                            1               5
```

<210> 28
<211> 10
<212> PRT
<213> Bos taurus

<220>
<221> MISC_FEATURE
<222> (1)..(10)
<223> Alp[ha S2 casein peptide

<400> 28

```
                    Thr Glu Glu Glu Lys Asn Arg Leu Asn Phe
                    1               5               10
```

<210> 29
<211> 14
<212> PRT
<213> Bos taurus

<220>
<221> MISC_FEATURE
<222> (1)..(14)
<223> Alpha S2 casein peptide

<400> 29

```
            Gln Gly Pro Ile Val Leu Asn Pro Trp Asp Gln Val Lys Arg
            1               5               10
```

<210> 30
<211> 14
<212> PRT
<213> Bos taurus

<220>
<221> MISC_FEATURE
<222> (1)..(14)
<223> Alpha S2 casein peptide

<400> 30

```
            Tyr Gln Gly Pro Ile Val Leu Asn Pro Trp Asp Gln Val Lys
            1               5               10
```

<210> 31
<211> 15
<212> PRT
<213> Bos taurus

<220>
<221> MISC_FEATURE
<222> (1)..(15)
<223> Alpha S2 casein peptide

<400> 31

```
            Tyr Gln Gly Pro Ile Val Leu Asn Pro Trp Asp Gln Val Lys Arg
            1               5               10              15
```

<210> 32
<211> 7
<212> PRT

<213> Bos taurus

<220>
<221> MISC_FEATURE
<222> (1)..(7)
<223> Beta casein

<220>
<221> MISC_FEATURE
<222> (1)..(7)
<223> Beta casein peptide

<400> 32

```
                            Gly Pro Phe Pro Ile Ile Val
                            1                   5
```

<210> 33
<211> 8
<212> PRT
<213> Bos taurus

<220>
<221> MISC_FEATURE
<222> (1)..(8)
<223> Beta casein

<220>
<221> MISC_FEATURE
<222> (1)..(8)
<223> Beta casein peptide

<400> 33

```
                            Thr Leu Thr Asp Val Glu Asn Leu
                            1                   5
```

<210> 34
<211> 11
<212> PRT
<213> Bos taurus

<220>
<221> MISC_FEATURE
<222> (1)..(11)
<223> Beta casein peptide

<400> 34

```
                        Gly Pro Val Arg Gly Pro Phe Pro Ile Ile Val
                        1               5                   10
```

<210> 35
<211> 12
<212> PRT
<213> Bos taurus

<220>

<221> MISC_FEATURE
<222> (1)..(12)
<223> Beta casein peptide

<400> 35

```
Thr Asp Val Glu Asn Leu His Leu Pro Leu Pro Leu
1               5                   10
```

<210> 36
<211> 13
<212> PRT
<213> Bos taurus

<220>
<221> MISC_FEATURE
<222> (1)..(13)
<223> Beta casein

<400> 36

```
Gln Glu Pro Val Leu Gly Pro Val Arg Gly Pro Phe Pro
1               5                   10
```

<210> 37
<211> 14
<212> PRT
<213> Bos taurus

<220>
<221> MISC_FEATURE
<222> (1)..(14)
<223> Beta casein

<400> 37

```
Tyr Gln Glu Pro Val Leu Gly Pro Val Arg Gly Pro Phe Pro
1               5                   10
```

<210> 38
<211> 14
<212> PRT
<213> Bos taurus

<220>
<221> MISC_FEATURE
<222> (1)..(14)
<223> Beta casein peptide

<400> 38

```
Ala Val Pro Tyr Pro Gln Arg Asp Met Pro Ile Gln Ala Phe
1               5                   10
```

<210> 39
<211> 16
<212> PRT
<213> Bos taurus

```
<220>
<221> MISC_FEATURE
<222> (1)..(16)
<223> Beta casein peptide

<400> 39


        Gln Glu Pro Val Leu Gly Pro Val Arg Gly Pro Phe Pro Ile Ile Val
        1                   5                   10                  15


<210> 40
<211> 17
<212> PRT
<213> Bos taurus

<220>
<221> MISC_FEATURE
<222> (1)..(17)
<223> Beta casein peptide

<400> 40


        Tyr Gln Glu Pro Val Leu Gly Pro Val Arg Gly Pro Phe Pro Ile Ile
        1                   5                   10                  15


        Val


<210> 41
<211> 19
<212> PRT
<213> Bos taurus

<220>
<221> MISC_FEATURE
<222> (1)..(19)
<223> Beta casein

<400> 41


        His Lys Glu Met Pro Phe Pro Lys Tyr Pro Val Glu Pro Phe Thr Glu
        1                   5                   10                  15


        Ser Gln Ser


<210> 42
<211> 20
<212> PRT
<213> Bos taurus

<220>
<221> MISC_FEATURE
<222> (1)..(20)
<223> Beta casein

<400> 42
```

```
Phe Gln Ser Glu Glu Gln Gln Gln Thr Glu Asp Glu Leu Gln Asp Lys
1               5                   10                  15

Ile His Pro Phe
            20
```

<210> 43
<211> 25
<212> PRT
<213> Bos taurus

<220>
<221> MISC_FEATURE
<222> (1)..(25)
<223> Beta casein

<400> 43

```
Ser Gln Ser Lys Val Leu Pro Val Pro Gln Lys Ala Val Pro Tyr Pro
1               5                   10                  15

Gln Arg Asp Met Pro Ile Gln Ala Phe
            20                  25
```

<210> 44
<211> 6
<212> PRT
<213> Bos taurus

<220>
<221> MISC_FEATURE
<222> (1)..(6)
<223> Kappa casein

<220>
<221> MISC_FEATURE
<222> (1)..(6)
<223> Kappa casein peptide

<400> 44

```
Tyr Ile Pro Ile Gln Tyr
1               5
```

<210> 45
<211> 6
<212> PRT
<213> Bos taurus

<220>
<221> MISC_FEATURE
<222> (1)..(6)
<223> Kappa casein peptide

<400> 45

```
                            Leu Pro Tyr Pro Tyr Tyr
                            1               5
```

<210> 46
<211> 7
<212> PRT
<213> Bos taurus


<220>
<221> MISC_FEATURE
<222> (1)..(7)
<223> Alpha S1 casein peptide


<400> 46

```
                       Ala Pro Phe Pro Glu Val Phe
                       1               5
```

<210> 47
<211> 11
<212> PRT
<213> Bos taurus


<220>
<221> MISC_FEATURE
<222> (1)..(11)
<223> Alpha S1 casein peptide


<400> 47

```
                  Ala Gln Gln Lys Glu Pro Met Ile Gly Val Asn
                  1               5                   10
```

<210> 48
<211> 12
<212> PRT
<213> Bos taurus


<220>
<221> MISC_FEATURE
<222> (1)..(12)
<223> Alpha S1 casein peptide


<400> 48

```
             His Ala Gln Gln Lys Glu Pro Met Ile Gly Val Asn
             1               5                   10
```

<210> 49
<211> 11
<212> PRT
<213> Bos taurus


<220>
<221> MISC_FEATURE
<222> (1)..(11)
<223> Alpha S1 casein peptide

<400> 49

```
            Ile Gln Lys Glu Asp Val Pro Ser Glu Arg Tyr
            1               5                   10
```

<210> 50
<211> 12
<212> PRT
<213> Bos taurus

<220>
<221> MISC_FEATURE
<222> (1)..(12)
<223> Alpha S1 casein peptide

<400> 50

```
            His Gln Gly Leu Pro Gln Glu Val Leu Asn Glu Asn
            1               5                   10
```

<210> 51
<211> 13
<212> PRT
<213> Bos taurus

<220>
<221> MISC_FEATURE
<222> (1)..(13)
<223> Alpha S1 casein peptide

<400> 51

```
            Ala Gln Gln Lys Glu Pro Met Ile Gly Val Asn Gln Glu
            1               5                   10
```

<210> 52
<211> 13
<212> PRT
<213> Bos taurus

<220>
<221> MISC_FEATURE
<222> (1)..(13)
<223> Alpha S1 casein peptide

<400> 52

```
            His Gln Gly Leu Pro Gln Glu Val Leu Asn Glu Asn Leu
            1               5                   10
```

<210> 53
<211> 14
<212> PRT
<213> Bos taurus

<220>
<221> MISC_FEATURE

<222> (1)..(14)
<223> Alpha S1 casein peptide

<400> 53

```
Ala Pro Phe Pro Glu Val Phe Gly Lys Glu Lys Val Asn Glu
1               5                   10
```

<210> 54
<211> 14
<212> PRT
<213> Bos taurus

<220>
<221> MISC_FEATURE
<222> (1)..(14)
<223> Alpha S1 casein peptide

<400> 54

```
Lys His Gln Gly Leu Pro Gln Glu Val Leu Asn Glu Asn Leu
1               5                   10
```

<210> 55
<211> 14
<212> PRT
<213> Bos taurus

<220>
<221> MISC_FEATURE
<222> (1)..(14)
<223> Alpha S1 casein peptide

<400> 55

```
Val Pro Gln Leu Glu Ile Val Pro Asn Ser Ala Glu Glu Arg
1               5                   10
```

<210> 56
<211> 15
<212> PRT
<213> Bos taurus

<220>
<221> MISC_FEATURE
<222> (1)..(15)
<223> Alpha S1 casein peptide

<400> 56

```
Ile Lys His Gln Gly Leu Pro Gln Glu Val Leu Asn Glu Asn Leu
1               5                   10                  15
```

<210> 57

<211> 15
<212> PRT
<213> Bos taurus

<220>
<221> MISC_FEATURE
<222> (1)..(15)
<223> Alpha S1 casein peptide

<400> 57

```
        Lys Val Pro Gln Leu Glu Ile Val Pro Asn Ser Ala Glu Glu Arg
        1               5                   10                  15
```

<210> 58
<211> 18
<212> PRT
<213> Bos taurus

<220>
<221> MISC_FEATURE
<222> (1)..(18)
<223> Alpha S1 casein peptide

<400> 58

```
        Ser Asp Ile Pro Asn Pro Ile Gly Ser Glu Asn Ser Glu Lys Thr Thr
        1               5                   10                  15

        Met Pro
```

<210> 59
<211> 16
<212> PRT
<213> Bos taurus

<220>
<221> MISC_FEATURE
<222> (1)..(16)
<223> Alpha S1 casein peptide

<400> 59

```
        Tyr Lys Val Pro Gln Leu Glu Ile Val Pro Asn Ser Ala Glu Glu Arg
        1               5                   10                  15
```

<210> 60
<211> 17
<212> PRT
<213> Bos taurus

<220>
<221> MISC_FEATURE
<222> (1)..(17)
<223> Alpha S1 casein peptide

<400> 60

```
Val Pro Gln Leu Glu Ile Val Pro Asn Ser Ala Glu Glu Arg Leu His
1               5                   10                  15

Ser
```

<210> 61
<211> 19
<212> PRT
<213> Bos taurus

<220>
<221> MISC_FEATURE
<222> (1)..(19)
<223> Alpha S1 casein peptide

<400> 61

```
Phe Ser Asp Ile Pro Asn Pro Ile Gly Ser Glu Asn Ser Glu Lys Thr
1               5                   10                  15

Thr Met Pro
```

<210> 62
<211> 17
<212> PRT
<213> Bos taurus

<220>
<221> MISC_FEATURE
<222> (1)..(17)
<223> Alpha S1 casein peptide

<400> 62

```
Lys Tyr Lys Val Pro Gln Leu Glu Ile Val Pro Asn Ser Ala Glu Glu
1               5                   10                  15

            Arg
```

<210> 63
<211> 18
<212> PRT
<213> Bos taurus

<220>
<221> MISC_FEATURE
<222> (1)..(18)
<223> Alpha S1 casein peptide

<400> 63

26

```
Lys Lys Tyr Lys Val Pro Gln Leu Glu Ile Val Pro Asn Ser Ala Glu
1               5                   10                  15

Glu Arg
```

<210> 64
<211> 20
<212> PRT
<213> Bos taurus

<220>
<221> MISC_FEATURE
<222> (1)..(20)
<223> Alpha S1 casein peptide

<400> 64

```
Arg Pro Lys His Pro Ile Lys His Gln Gly Leu Pro Gln Glu Val Leu
1               5                   10                  15

Asn Glu Asn Leu
            20
```

<210> 65
<211> 5
<212> PRT
<213> Bos taurus

<220>
<221> MISC_FEATURE
<222> (1)..(5)
<223> Alpha S2 casein peptide

<400> 65

```
Ala Leu Pro Gln Tyr
1               5
```

<210> 66
<211> 7
<212> PRT
<213> Bos taurus

<220>
<221> MISC_FEATURE
<222> (1) .. (7)
<223> Alpha S2 casein peptide

<400> 66

```
Ala Val Pro Ile Thr Pro Thr
1               5
```

<210> 67
<211> 14

<212> PRT
<213> Bos taurus

<220>
<221> MISC_FEATURE
<222> (1)..(14)
<223> Alpha S2 casein peptide

<400> 67

```
Gly Pro Ile Val Leu Asn Pro Trp Asp Gln Val Lys Arg Asn
1               5                   10
```

<210> 68
<211> 14
<212> PRT
<213> Bos taurus

<220>
<221> MISC_FEATURE
<222> (1)..(14)
<223> Alpha S2 casein peptide

<400> 68

```
Gln Gly Pro Ile Val Leu Asn Pro Trp Asp Gln Val Lys Arg
1               5                   10
```

<210> 69
<211> 15
<212> PRT
<213> Bos taurus

<220>
<221> MISC_FEATURE
<222> (1)..(15)
<223> Alpha S2 casein peptide

<400> 69

```
Gln Gly Pro Ile Val Leu Asn Pro Trp Asp Gln Val Lys Arg Asn
1               5                   10                  15
```

<210> 70
<211> 15
<212> PRT
<213> Bos taurus

<220>
<221> MISC_FEATURE
<222> (1)..(15)
<223> Alpha S2 casein peptide

<400> 70

```
Tyr Gln Gly Pro Ile Val Leu Asn Pro Trp Asp Gln Val Lys Arg
1               5                   10                  15
```

<210> 71
<211> 16
<212> PRT
<213> Bos taurus

<220>
<221> MISC_FEATURE
<222> (1)..(16)
<223> Alpha S2 casein peptide

<400> 71

```
Tyr Gln Gly Pro Ile Val Leu Asn Pro Trp Asp Gln Val Lys Arg Asn
1               5                   10                  15
```

<210> 72
<211> 7
<212> PRT
<213> Bos taurus

<220>
<221> MISC_FEATURE
<222> (1)..(7)
<223> Beta casein peptide

<400> 72

```
Thr Leu Thr Asp Val Glu Asn
1               5
```

<210> 73
<211> 10
<212> PRT
<213> Bos taurus

<220>
<221> MISC_FEATURE
<222> (1)..(10)
<223> Beta casein peptide

<400> 73

```
Ala Val Pro Tyr Pro Gln Arg Asp Met Pro
1               5                   10
```

<210> 74
<211> 12
<212> PRT
<213> Bos taurus

<220>
<221> MISC_FEATURE
<222> (1)..(12)
<223> Beta casein peptide

<400> 74

```
Lys Glu Met Pro Phe Pro Lys Tyr Pro Val Glu Pro
1               5                   10
```

<210> 75
<211> 13
<212> PRT
<213> Bos taurus

<220>
<221> MISC_FEATURE
<222> (1)..(13)
<223> Beta casein peptide

<400> 75

```
Ala Val Pro Tyr Pro Gln Arg Asp Met Pro Ile Gln Ala
1               5                   10
```

<210> 76
<211> 13
<212> PRT
<213> Bos taurus

<220>
<221> MISC_FEATURE
<222> (1)..(13)
<223> Beta casein peptide

<400> 76

```
His Lys Glu Met Pro Phe Pro Lys Tyr Pro Val Glu Pro
1               5                   10
```

<210> 77
<211> 14
<212> PRT
<213> Bos taurus

<220>
<221> MISC_FEATURE
<222> (1)..(14)
<223> Beta casein peptide

<400> 77

```
Ala Val Pro Tyr Pro Gln Arg Asp Met Pro Ile Gln Ala Phe
1               5                   10
```

<210> 78
<211> 16
<212> PRT
<213> Bos taurus

<220>
<221> MISC_FEATURE
<222> (1)..(16)

<223> Beta casein peptide

<400> 78

```
        Gln Glu Pro Val Leu Gly Pro Val Arg Gly Pro Phe Pro Ile Ile Val
        1               5                   10                  15
```

<210> 79
<211> 17
<212> PRT
<213> Bos taurus

<220>
<221> MISC_FEATURE
<222> (1)..(17)
<223> Beta casein peptide

<400> 79

```
        Tyr Gln Glu Pro Val Leu Gly Pro Val Arg Gly Pro Phe Pro Ile Ile
        1               5                   10                  15

        Val
```

<210> 80
<211> 17
<212> PRT
<213> Bos taurus

<220>
<221> MISC_FEATURE
<222> (1)..(17)
<223> Beta casein peptide

<400> 80

```
        Lys Lys Ile Glu Lys Phe Gln Ser Glu Glu Gln Gln Gln Thr Glu Asp
        1               5                   10                  15

        Glu
```

<210> 81
<211> 19
<212> PRT
<213> Bos taurus

<220>
<221> MISC_FEATURE
<222> (1)..(19)
<223> Beta casein peptide

<400> 81

```
His Lys Glu Met Pro Phe Pro Lys Tyr Pro Val Glu Pro Phe Thr Glu
1               5               10              15

Ser Gln Ser
```

<210> 82
<211> 19
<212> PRT
<213> Bos taurus

<220>
<221> MISC_FEATURE
<222> (1)..(19)
<223> Beta casein peptide

<400> 82

```
Lys Lys Ile Glu Lys Phe Gln Ser Glu Glu Gln Gln Gln Thr Glu Asp
1               5               10              15

Glu Leu Gln
```

<210> 83
<211> 24
<212> PRT
<213> Bos taurus

<220>
<221> MISC_FEATURE
<222> (1)..(24)
<223> Beta casein peptide

<400> 83

```
Ser Gln Ser Lys Val Leu Pro Val Pro Gln Lys Ala Val Pro Tyr Pro
1               5               10              15

Gln Arg Asp Met Pro Ile Gln Ala
            20
```